# EUROPEAN PATENT APPLICATION

(11) **EP 1 221 671 A2**
(43) Date of publication of application: **10.07.2002**
(21) Application number: 01104070.6
(22) Date of filing: 20.02.2001
(51) Int. Cl.: G06F 19/00

(54) **Method for organizing and depicting biological elements**

(30) Priority: 05.01.2001 EP 01100362
(71) Applicant: LION Bioscience AG, 69120 Heidelberg (DE)
(72) Inventor: O'Donoghne, Séan, D-69117 Heidelberg (DE); Fries, Karsten, D-69214 Eppelheim (DE)

(57) **Abstract**

The present invention for the first time provides for a method for organizing and depicting one or more biological elements stemming from one or more databases and one or more data sets whereby at least a first feature element is determined from each of said data sets, a graphical representation is made for displaying the biological elements corresponding to said data sets and a display of a basic environment element is enhanced by locating at least one of said graphical representations of said biological elements on the display depending on the information contained in the feature element.

## Description

### BACKGROUND OF THE INVENTION

A major challenge in the life sciences today is dealing with the rapid growth in biological, chemical and/or medical data; the best known examples are the DNA and/or protein sequence databases, which are increasing in number and content size exponentially; more rapidly, in fact, than the increase in computer speed. In addition, other methods such as protein structure determination and mass spectroscopy are being scaled up for high-throughput; this is creating a corresponding increase not just in the size, but the number of biological databases available each year. To deal with this rapid increase in data, we require informatics tools that can automatically organize new and existing data, and allow the user to maintain an overview.

The most successful and best known method for integrating biological chemical and/or medical data databases is to construct a 'meta'-database that keeps track of the main databases as they grow, adding cross-links between the databases. With such systems, a user can submit queries and get back a formatted text summary with links to matching entries in any of the original biological databases. Two well known systems of this type are SRS ('Sequence Retrieval System', a product distributed by LION Bioscience AG) and the Entrez server (www.ncbi.nlm.gov/entrez). As with text-based computer operating systems (e.g., MS-DOS or Unix), such systems are ideal for expert users, but for non-experts they have a number of draw-backs. To make these systems amenable to non-experts, graphical user interfaces (GUIs) help the user navigate through the data.

To date, most tools for visualizing biological databases are designed to show essentially only one type of database. Methods that can combine different types of data into a single view are desired. One important example of combining different data is mapping of functional properties (single nucleotide polymorphisms or SNPs, sequence conservation, residues involved in protein-protein interaction, and active sites) onto the protein 3D structure. Such combined views could then be visualized using, e.g. standard molecular graphics methods such as RasMol (www.umass.edu/microbio/rasmol).

It would be very desirable however to have a method for organizing and depicting one or more biological elements; preferentially molecules, stemming from one or more databases and one or more data sets whereby at least a first feature element is determined from each of said data sets, a graphical representation is made for displaying the biological elements corresponding to said data sets and a display of a basic environment element is enhanced by locating at least one of said graphical representations of said biological elements on the display depending on the information contained in the feature element.

The present invention for the first time provides for such a method.

### DETAILED DESCRIPTION OF THE INVENTION

The present invention provides for a method for organizing and depicting one or more biological elements comprising the steps of, receiving an input to select one or more databases, receiving further input to select one or more desired data sets from the one or more databases based on one or more selection criteria, determining at least one first feature element from each of said one or more data sets, determining a graphical representation for displaying at least one of the biological elements corresponding to said one or more data sets and, enhancing a display of a basic environment element by locating at least one of said graphical representations of said biological elements on said display of said basic environment element, depending on the information contained in said first feature element.

A biological element may be a compound, a molecule, regardless of whether they are of organic or inorganic nature. In a preferred embodiment the biological element is an element that may be found in living organisms, such as a protein, a nucleic acid, large macromolecular complexes, such as ribosomes or the like but the invention is not limited to these. Also encompassed by this description are, e.g. pharmaceutical compounds or lead molecules which may be found in various databases. The invention also encompasses compounds which interact with molecules of a living organism to be biological elements. Such biological elements may be herbicides, pesticides or the like. One skilled in the art will recognize many different kinds of biological elements all of which should primarily either be present in a living organism or interact with a living organism or a part thereof.

The invention makes use of one or more databases herein a database may be for example, a flat-file database, or a relational database or an XML database. Commonly used databases for the foregoing invention are those which contain information on biological elements. Examples are SWISS-PROT, BIND, PDB, INTERPRO, GENBANK, and EMBL.. The databases may present within the system that runs the method according to the invention or located externally.

The data sets are chosen based on one or more selection criteria. Such criteria may be the origin from which the biological element stems to which the data set pertains, *e.g* one may "choose all proteins known in humans". Or one may "select all proteins that are present in the nucleus of a rat cell". Or one may "select all human proteins that are related to cancer". Any given criteria may be applied for selecting the data sets and thus the biological elements.

When the method according to the invention is applied, a first feature element is determined for each of the data sets. The feature element represents a piece of information that characterizes the element in further detail and is needed to place the graphical representation within the display of a basic environment element. So in one embodiment for example data sets pertaining to human proteins are extracted from a database. The first feature element that is determined pertains to the information within the data set, which defines the localization of the proteins in a cell. This could be in the nucleus for example (see also figures). In a preferred embodiment this is done by extracting the sub-cellular location from, e.g. a SWISS-PROT entry, or from an entry in, e.g. the BIND database. For some biological elements, the sub-cellular location is not available in a database. In this case, the sub-cellular location can be inferred by applying a method for calculating properties such SignalP (Nielsen, H., J. Engelbrecht, et al. (1997). Protein Engineering **10:** 1-6.) and/or PreLoc (Andrade, M. A., S. I. O'Donoghue, et al. (1998) Journal of Molecular Biology **276:** 517-525). These predictions can also be precalculated and provided as a database.

Based on the first feature element, that biological element is placed into the space of basic environment. The basic environment may consist of a graphic chosen from the group comprising an organism, one or more tissue types, a cell, an organelle, a sub-cellular compartment, a molecule, an atom and/or a sub atomic particle. It may also comprise more than one of the above. In preferred embodiments of the invention it is a cell image. In preferred embodiments the different cell types which are possible are created separately; here in the figures, the inventors have chosen a generalized eukaryotic cell. The cell membranes may be e.g. defined using standard vector graphics methods, such as postscript curves; the curves are initially defined based on images of the cell topology from microscope images of cells. This is done either by hand or by fitting mathematical parametric curves to images of the membranes from cell micrographs. A background bitmap image is then constructed with different shadings to give more richness to the image, and help the user differentiate between different parts of the cell.

In a preferred embodiment of the invention the method according to the invention makes use of molecules as biological elements. In this embodiment, e.g. proteins, nucleic acids, fatty acids, carbohydrates, peptides or the like are displayed. In one embodiment molecules which are found within living organisms are displayed together with those that are not known to be found within living organisms such as, e.g. pharmaceutical compounds. Hence, it is possible to analyze and/or depict their possible interaction. In a particularly preferred embodiment of the invention proteins are displayed.

In one embodiment of the method for organizing and depicting one or more biological elements according to the invention the display of said basic environment element is enhanced by locating at least 50 of said graphical representations of said biological elements on said display of said basic environment element. It is equally possible locate at least 500 of said graphical representations of said biological elements on said display of said basic environment element and it is particularly preferred to locate at least 5000 of said graphical representations of said biological elements on said display of said basic environment element.

In one embodiment all proteins known to be in a given cell type of one organism are depicted as biological elements according to the invention and the display of said basic environment element in this case a cell is enhanced by locating all of said graphical representations of said biological elements on said display of said basic environment element.

The method according to the invention makes it possible to add or subtract any of the biological elements to and from the display of said basic environment element at any given time. It is also possible to display different subsets of biological elements simultaneously by, for example by coloring the elements with different colors. Thus, it is possible to choose subsets by any criteria (such as an SRS query) and display the results of these queries.

Ordinarily one or more further feature elements are determined for said one or more data sets and the one or more further feature elements are extracted from the same database as the database from which the first feature element was determined. It is however just as likely that one or more further feature elements are determined for said one or more data sets and the one or more further feature elements are extracted from one or more different databases as the database from which the first feature element was determined.

So in a given example, human proteins are located within the virtual cell based on the information from the first feature element. The relationship, *i.e.* the binding of two or more proteins to each other, or the spatial closeness and/or distance amongst the proteins is depicted based on information stemming from a different database, that may for example contain experimental data from Yeast-Two-Hybrid experiments (methods such as described by Fields and Song *Nature* 340, pp245 (1989), Bartel et al., *Biotechniques* 14, pp920 (1993) and Lee et al. Nature 374 pp91-4 (1995)). It may also contain experimental data concerning macromolecular complexes determined either from mass spectroscopy or from X-ray crystallography. It may also contain data about protein interaction that is inferred or predicted by methods such as homology inference or protein-protein docking algorithms. Thus, in this preferred embodiment the second feature element helps organize the proteins in a biologically meaningful way. Whereas the first feature element also accomplished that, the intent there is to organize the biological elements with respect to their localization within the virtual cell. It is the intent of the second feature element to organize the proteins with respect to one another.

Thus, in one embodiment of the method according to the invention an additional step of, determining at least one second feature element for said at least one data set and, further enhancing the display of said basic environment element by using the information from said second feature element to place the at least one biological element into said basic graphical representation of said basic environment element is performed.

In a further embodiment a further feature element for said at least one data set is determined however it is used to further enhance the display of said at least one graphical representation of said biological element.

The inventors have made use of this embodiment of the invention, e.g. enhancing the image of proteins when shown within a cell. Here, information is extracted from the PDB database of three dimensional structures of biomolecules. Based on the area of the display of the basic environment element that is chosen, *i.e.* a close view or a distant view, it is possible to represent proteins as either 'zero'-dimensional single points, as one-dimensional strings or graphical objects, as two-dimensional images, or as three dimensional objects.

The exact 3D structure is presently only known for around 50% of known proteins. For about 30% of the proteins with unknown 3D structure, the 3D structure can be inferred by homology to one or more known structures. The inferred structure can be used as the best current model of the 3D structure. For around 20% of proteins, no precise details of the structure are available. For these proteins, some aspects of structure can still be inferred by prediction methods, such as secondary structure. The various information about the structure, predicted or experimental, can be combined into a 3D model. In the preferred embodiment, these model are represented in such a way as to make it clear that the 3D information is only inferred. This representation is chosen to be clearly different from that used to represent biological elements where the exact 3D is known.

Similarly, exact 3D structure of chromosome is not known at atomic detail, however in the preferred embodiment, models of the chromosome structures are built and used.

A further feature element for said at least one data set pertaining to said at least one biological element may contain information pertaining to one of the activities of said biological elements, thus the feature element may be used to enhance the display of said at least one graphical representation of said biological element by displaying the activity thereof. The inventors have used this feature of the invention to display, e.g. enzymatic activities. In this embodiment, e.g. a kinase enzyme may be displayed during the action of phosphorylating another protein. The feature element in this particular case is the entry within the data-set pertaining to the description of the enzymatic activity. Feature elements may also be used to, e.g. display the "life-time", i.e. time of presence of a biological elements or other aspects pertaining to the fate of such an entity. Feature elements may also be used to enhance the display by depicting other physical or chemical properties of a biological element.

In some embodiments of the invention one or more databases, are chosen from the group comprising databases that comprise data sets with information regarding biomolecules, organic molecules and/or inorganic molecules found in living organisms. In preferred embodiments the databases comprise data sets with information regarding genes and/or proteins.

The basic environment element is often a representation chosen from the group comprising an organism, one or more tissue types, a cell, an organelle, a sub-cellular compartment, a large complex of macromolecules, a molecule, an atom and/or a sub atomic particle.

In a preferred embodiment it is a eukaryotic, or prokaryotic, or archean cell or a virus particle.

It is of course possible to depict any of the elements either in a zero-dimensions (single points), one dimension, two dimension,alor three dimensions.

In one embodiment the area that is depicted in said display of said basic environment element is selected by choosing an area in a second display of said basic environment element.

The inventors have done this for the eukaryotic cell. Here, one display shows the entire cell as well as a smaller window therein, which when moved, changes the area of the cell displayed in a second display (see also figures).

The invention also pertains to a display of a basic environment element, obtainable by a method according to the invention especially a computer display. The invention also pertains to a data structure representing a graphic display, said graphic display being obtainable by applying the method according to the invention. In one embodiment at least 50 of said graphical representations of said biological elements are presented, in another embodiment at least 500 of said graphical representations of said biological elements are present, in the most preferred embodiment at least 5000 of said graphical representations of said biological elements are presented.

The invention also concerns a computer readable medium for embodying or storing therein data readable by a computer, said medium comprising one or more of the following, a data structure generated by executing a method according to any of the embodiments of the invention, computer program code means which is adapted to cause a computer to execute a method according to any of the embodiments of the invention.
The invention also concerns a system for enabling different users to access and share common data structures associated with the method. The access may be either locally, within an organization, or may be distributed remotely, for example, via the internet.

The invention also concerns an apparatus for organizing and depicting one or more biological elements by applying the method according to the invention.

### DETAILED DESCRIPTION OF PREFERRED EMBODIMENTS

In preferred embodiments the foregoing invention is also able to solve the following problems for the first time.

The sub-cellular location of a protein is one of the most fundamental functional properties. There are increasing more data that accurately define where in the cell proteins occur; however, no available method is able to visualize such data, either for a single protein, a selection of many proteins, or for one or more entire genomes. The invention enables the user to rapidly see the subcellular distribution of a selected group of proteins.

By combining together information from several different databases and presenting the result in a single view, the invention organizes and depicts these data into a simpler, more intuitive form.

A major initial hurdle in learning to understand complex data sets in particular biochemical data sets is to develop an overview of the processes involved. This is difficult primarily because of the large number of different data types or *e.g*. proteins and reactions involved. The invention is a highly interactive thus, an intuitive tool that encourages the user to explore, and also provides an immediately comprehensible context of where each data set fits into a broader picture.

The invention can be used to view periodic database queries relevant to a particular topic. However, this unique visual arrangement of results provides a different kind of 'filtering' that may sometime lead to significant new insight. For example, a researcher may use the invention to check for new experimental finding about his favorite data set of genes or proteins by periodically starting the invention and zooming straight to that data set or protein. When a significant new result has become available (e.g. a new SNP site in a neighboring protein), the user will immediately see the difference. By contrast, with database approaches, the user will generally only find data that she/he specifically requests through a query.

With the explosion in data in particular in the field of biology and chemistry *e.g.* protein sequences and structures, it becomes increasingly difficult to keep track of particular information pertaining to data sets as *e.g*. proteins of interest.

Currently, one needs to remember the name or accession number of a protein. In contrast, a user of the invention in a preferred embodiment can *e.g.* find proteins he is interested in, based on their location in the 2D view.

The details of the cell view (membranes, large complexes of proteins and lipids) provide a memory 'anchor' or a context - once one has seen the cellular context in which a protein occurs, it is very easy to remember where the protein lies in the view. Thus, at a later stage, one can simply and quickly return to the part of the cell to find the protein again. This is a completely novel way of finding information about and/or within data in particular proteins, and has great advantages over the traditional database approach known in the prior art.

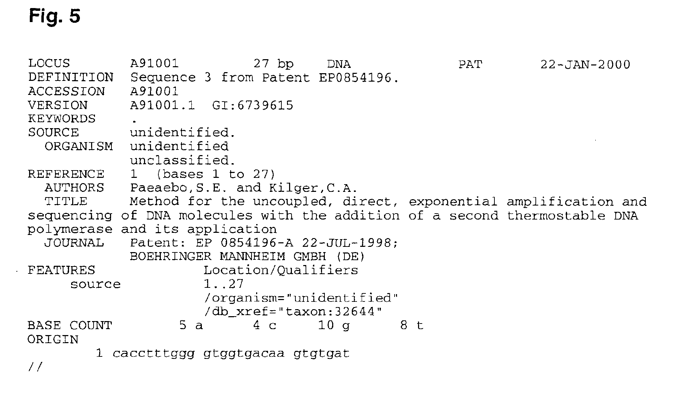

Most people are much better at remembering spatial locations than at remembering the strings of numbers and characters that are used for database accession numbers. An example of a database entry is shown in Fig. 5.

The mnemonic feature of the invention results from the context created for each protein i.e., the location in the cell, the proximity to other cellular structures and other proteins, and also the shape of the proteins structures and the appearance of the annotations mapped on the structures. All of these provide the mnemonic 'anchors' that will help the user recall not just the location of a given protein, but also the location of abstract annotations.

For example, a protein with unusually highly conserved sequence may be colored completely one color, such as blue. The user may then remember having seen fully blue protein at a particular location, for example close to a large complex of proteins and close to the mitochondrial outer membrane; thus, he would be able to find the protein again without having to remember its name.

Visualization of 3D structures of membrane proteins are a special case as they are among the most important medically, but unfortunately very few 3D structures of membrane proteins are known. There is currently no method for automatically modeling the membrane topology, placing this within a membrane, and mapping onto the model important functional properties. Such a view by itself can give a useful insight into the protein function.

In a preferred embodiment in which the invention is applied to a biological question the invention defines a single view that combines information from several different databases, especially data on where proteins occur in the cell, data on which proteins form complexes, and data on the 3D structure of proteins. The view may consist of a 2D (or 3D) model of the cell; the proteins of interest are then placed into the subcellular compartments where they are known to occur.

Within each compartment, proteins that are known to bind together are placed together. The user may initially see an image of the whole cell with small proteins represented as single pixels on the computer monitor; very large macromolecular complexes such as chromosomes and ribosomes are represented as simplified 3D representations that hide the atomic detail, or as 2D images.

The user can then select a region and zoom into it. The user can zoom continuously from the cellular scale (micrometers) down to the molecular scale (nanometers); as the user zooms in on a protein it changes continuously from a single point at the micrometer scale (for small proteins) to a detailed 3D structure at the molecular scale. Where several proteins are known to interact, and where the interaction interfaces are known or can be predicted, these protein structures are positioned and aligned to show the exact interaction. In addition to representing the physical structure of each protein, the structure is annotated by coloring and by choice of representation to display other important aspects of protein function, such as protein family assignment, short sequence signature patterns, SNPs (single nucleotide polymorphisms) sites, sequence conservation, bound ligands, and active sites.

In a preferred embodiment of the invention the method enables the user to look in detail at regions of interest (either individual protein structure or a large aggregate or cluster of proteins), while maintaining an overview of where each region fits into the context of the whole cell.

The view is not necessarily trying to depict the actual physical arrangement of every single molecule in the cell; similarly, a geographical atlas does not try to model every tree or rock. Rather, the aim is visualize representative subsets of proteins that cover all interaction possibilities.

The invention provides a novel way to overview an entire genome of data, gaining at a quick glance an idea of how proteins are distributed in the cell.

The invention also provides a novel way of viewing selected subsets of an genome. For example, the invention could be used to view all human proteins for which the associated database entries have been added or update in the last month. Or the invention could be used to show only those proteins associated with a particular disease.

The invention provides a novel view of a large number of atomic resolution 3D structures of proteins.

The invention enables the visualization of another kind of information, namely the detail of the interacting surfaces, in addition to the cellular context of the interaction.

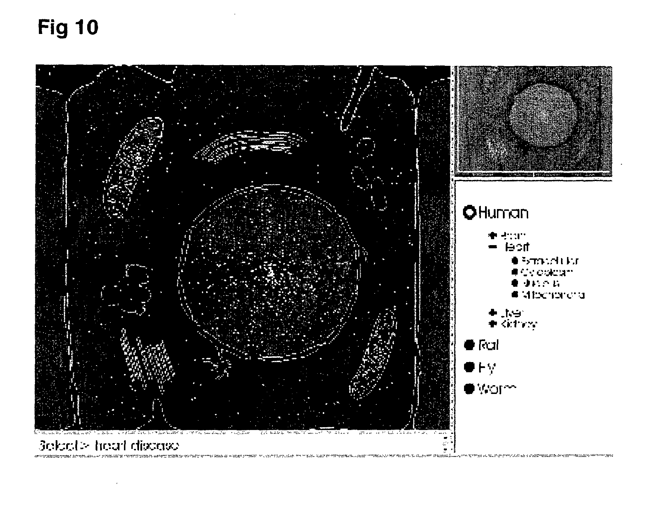

The invention can also be used as a simple molecular graphics tool for single proteins: when used in this way, it has several advantages compared with standard molecular graphics tools. Firstly, the subcellular location is made clear by the overview frame (top right, Figure 10), and by the ability to zoom quickly out to the cell view. The second major advantage is the immediate realization of which proteins are in vicinity or interacting with the particular protein of interest. Finally, given that neighboring proteins are present, the invention view can also display insightful representation of the inter-protein interactions through visualizing interaction surfaces or electrostatic potentials.

The invention can be extended to display tissues, organs, organisms or different cell types, such as neurons, plant cells, bacteria cells, and viruses.

The data placing in particular protein placing method can be extended or replaced by similar methods; other types of protein-protein interaction data may be used, such as metabolic or signaling pathway data.

The main view can be 3D; this can be visualized either using a standard PC monitor plus stereo glasses, or with 'immersion'-graphics systems.

The invention can be extended to include dynamics, this will facilitate the visualization of reaction data as well, for example letting the proteins move or 'diffuse' to different locations. Then, the invention would incorporate yet another type of databases (reactions or metabolic pathways) into its single view.

Data obtained from expression profiles analysis (DNA-chips or protein chip experiments) can also be included using static or dynamic methods.

Process such as protein expression and translocation, or signal transduction, can also be visualized in the invention.

A clear extension is to connect the viewer to a viewer of tissue and organism, i.e. to see the cells in their relationship to each other, each tissue type, and then the distribution of tissues within the body. Visualization of tissues within the body is a prior art, however, once again the inventive aspect here is to combine this view with a subcellular location view, and also a molecular graphics view. This also extends the field of application to medicine and health.

The view can be also extended to see even greater subatomic detail of parts of the protein or other molecules.

The method can be extended to view other macromolecules such as lipid aggregates and RNA; small molecules, such as ATP, can also be incorporated.

The invention can be extended to display different stages of the cell cycle, and also the show a simulation of the cell cycle.

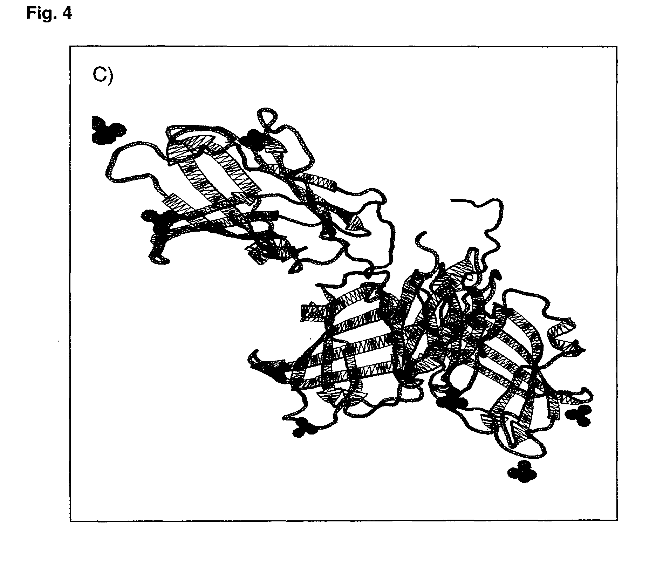

Embodiments of the present invention may be implemented using a computer system as schematically illustrated in Fig. 4. A computer system 600 may comprise a computer 605 connected to a display 610, a mouse 620, and comprising some storage medium 630 such as a floppy disk drive, a CD-ROM drive or the like, and some hardware components 640 comprising at least one CPU and a memory such as to enable the computer to carry out by means of the CPU program instructions stored in said memory. The program itself may be stored on any computer readable medium, or it may by carried out on a remote computer (host) to be accessed by a client computer through a communications link such as the internet. Hybrid implementations are also possible, such as a Java implementation being downloaded in part or as a whole through a network and carried out on a client.

### EXAMPLES

### EXAMPLE 1:

An initial selection of proteins is made by the user with a search engine such as SRS (Sequence Retrieval System). The example used here for illustrative purposes is all publicly available human sequences.

The proteins are then placed in their respective sub-cellular locations. For many proteins, the sub-cellular location are found in the corresponding entry in the SWISS-PROT database (www.expasy.ch/sprot) by querying the database through SRS. For those proteins with no annotated location, the location can be predicted either by homology to proteins with known location, or other predictive methods, such as those based on the presence of transmembrane helices (e.g., PHD, Rost 1996), of signal peptides (e.g. SignalP, Nielsen, Engelbrecht et al. 1997), or of characteristic amino acid composition (e.g., PreLoc, Andrade, O'Donoghue et al. 1998).

Membrane proteins are placed along the respective membranes, initially randomly. The position of the membrane proteins is then optimized such that proteins within the same membrane that are known to bind to each other are moved together: a repulsive term is also added to ensure than proteins known not to interact do not occur together in the final optimized positions. During the optimization, the proteins are constrained to lie always on the membrane.

Next, proteins are placed initially randomly into the spaces (nucleoplasm, cytoplasm, mitochondrial matrix, extracellular space, etc.), with a check to ensure that they are in the correct space. The positions of the non-membrane bound proteins are then optimized such that the proteins that are known to interact are moved together, those that do not are forced apart, and during the optimization, a constraint is added to ensure the proteins remain in the correct compartment.

Information about which proteins form complexes is extracted either from SWISS-PROT or other databases, such as BIND, that specifically annotate protein-protein complex formation. Further information about protein-protein binding can be predicted either by homology to proteins known to form complexes, or other predictive methods, such as protein-protein docking.

Information about protein 3D structure is then extracted from for example the PDB database; for proteins with no experimentally determined 3D structure, the 3D structure can often be inferred via homology modeling or threading methods.

One may also predict aspects of protein structure, such as solvent accessibility and secondary structure, using methods such as PHD.

Transmembrane helices can be reliably predicted; hence a two-dimensional representation can be built and placed in the respective membrane.

Proteins for which no 3D structural data is available are represented as 2D objects, or very flat 3D objects, to distinguish them from 3D structures.

The speed at which placing proteins into the view can be made very fast by precalculating placements; predictions of subcellular location, binding partners, and 3D structures can also be precalculated and stored on a centralized computer.

Access to these data are managed by server running on this machine.

### EXAMPLE 2:

### Displaying the View

The invention view is generated by e.g. a client program running on the users' machine. In a preferred embodiment one window with four separate frames is created the main frame (Fig. 10) shows the current region of interest - initially, this would usually be the whole cell. In this frame, the user can select a region with the mouse to zoom into, or can zoom out back to the whole cell view. The user can also select individual proteins, or groups of proteins, by selecting a region.

A second frame (top right, Fig. 10) always shows the whole cell view, and has lines (in green) indicating the region that is currently seen in the main frame.

A third frame (bottom right, Fig. 10) displays textual information about the currently selected group of proteins, such as the number of molecules selected, their names, amino acid sequence, etc.

A fourth frame (bottom left, Fig. 10) gives a command line interpreter where the user can enter queries to narrow the selection to a group of proteins matching the query.

### EXAMPLE 3:

The Basic Environment Element is a Cell or a Sub-Compartment Thereof:

In this preferred embodiment of the basic environment element the method and/or the system according to the invention makes use of a cell image bank which is broken up into elementary images corresponding to components of the main cell image.

These elements also form part of the image bank and can be used to carry out various images of cells. For example the nucleus is a basic element which will be useful for several type of cells.

For example, a cell is represented with the membrane, the nucleus, the cytoplasm and the principal cellular organelles (mitochondrion, golgi apparatus, chloroplast for the plant cells).

The cell image may contain the standard sub-cellular compartments. For example, a generalized eukaryotic cell will contain a cytoplasm,
mitochondrion, golgi apparatus, peroxysome, , and endoplasimic reticulum.
For specialized cells, such as nerve cells, can also be represented, in which case appropriate specialized structures, such as the axon can be represented.

### EXAMPLE 4:

Zooming into the view of the basic environment element:

In one embodiment of the invention as outlined above, the forgoing invention makes use of either an organism, one or more tissue types, a cell, an organelle, a sub-cellular compartment, a molecule, an atom and/or a sub atomic particle as a basic environment element. An organism may be up to meters in size ("m"), a particular tissue type is routinely in the millimeter size range ("mm"), an average cell is usually about a micrometer in size ("µm"), an organelle routinely a fraction thereof in size, a molecule is within the general dimension of nanometers. Atoms are in the range of 100s of picometers. Subatomic particles are much smaller.

In this preferred embodiment of the invention the basic environment element is a either an organism or a tissue, or a cell. Thus, the first view of the basic environment element displayed in accordance with the method according to the invention is, when zoomed out an area representing meters to millimeters. In this embodiment of the invention, the area displayed by the basic environment element may be changed in a continuos fashion in order to enable the "zooming in" starting from an area in the range of millimeters down to an area of picometers or even smaller.

Thus, a user of the method according to the invention would be able to select biological elements that are to be displayed on the basic environment element and follow their location, distribution and/or interaction within a tissue or cell down to the location, distribution, and/or interaction and more importantly physical characteristics within a picometer range. While changing the "view" the databases according to the invention are queried in order to determine desired feature elements which are needed to provide for the different displays within the basic environment element.

Thus, while no feature element is necessary for depicting the physical property of a protein at the mm level, the method must determined one or more at the picometer level, if the user desires to investigate the physical properties of the proteins at this level.

In this embodiment of the invention it is now possible to zoom in and out of an organism, down to the picometer range, while at the same time the necessary feature elements are determined both for the environment element as well as for the biological elements in order to make it possible to create "a continuos travel" through the dimensions, starting from meters travelling down to picometers. The invention makes it possible at the same time to, both chose and update the kind of biological elements that are displayed. In particular the data sets can be chosen from internal (part of the apparatus) and external (e.g. found within the internet) data bases which thus potentially provide for instant and up-to-date data sets.

Thus, in this embodiment of the invention a user may chose to analyze the relationship between a new set of proteins discovered and published at a given day and another particular set of proteins the user knows from the past by choosing the respective databases and/or data sets.

At the whole cell view, almost all proteins (the biological elements in this embodiment) are too small to be seen, and hence are represented as single pixels.

As the user zooms into a region at intermediate scale, such as the region in the nucleus, most proteins would still be too small to see and hence the representation for these proteins remains as single pixels.

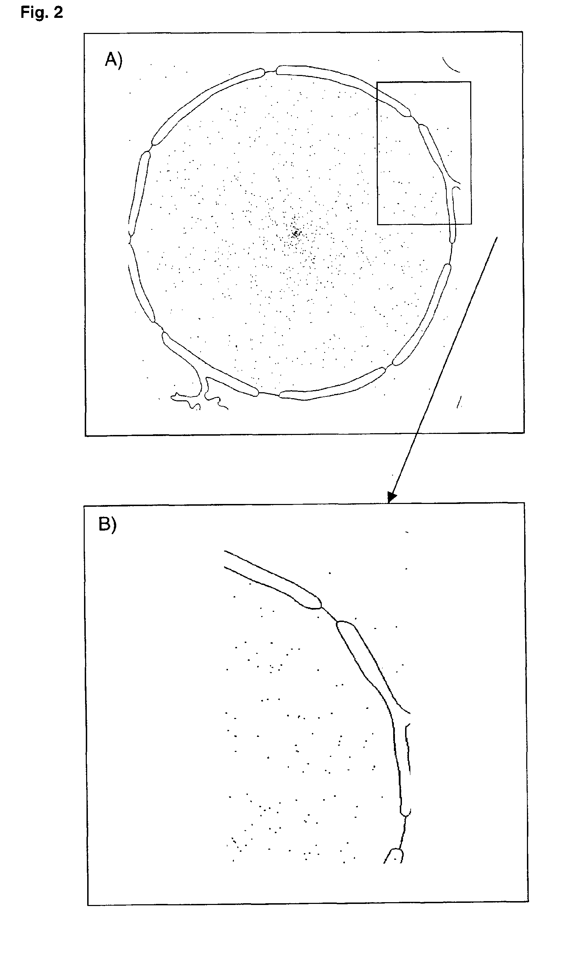

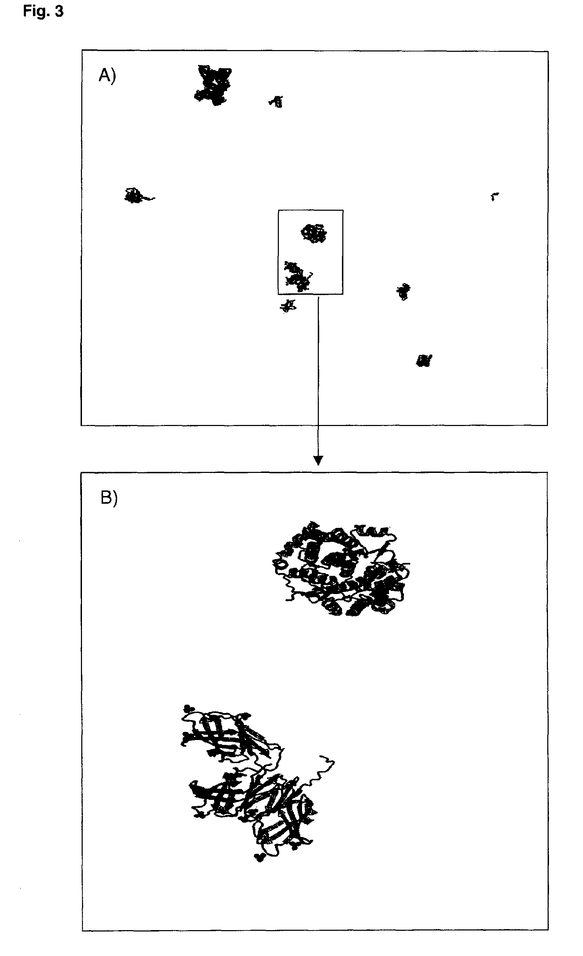

Once the user has zoomed in far enough such that the size of the protein becomes larger than a single point, the representation of the protein may be changed to provide more detail about the protein or biological element. This more detailed representation may be the full atomic-detail 3D structure, or a reduced 3D representation that hides atomic detail, or a 2D image of the protein structure (see Figures 2 & 3). Reduced representations would be important for allowing large macromolecular complexes, such as entire chromosomes or ribosomes, to be represented in a simple way. Thus, the local computer that manages the display will only need to deal with the reduced level of detail. In the preferred embodiment, when the user of the invention would like to examine one area of the complex or biological element in detail, the user zooms into that region and only at that particular time are the full details of that region transferred to the local computer from a server computer.

In the preferred embodiment of the invention, several different reduced representations are used between the single-point per protein and the full atomic detail 3D structure. For 3D reduced representations, progressively more detail is added to the 3D structure. For 2D reduced representations (images) several 2D images may be used with several different levels of resolution. Initially, the lowest resolution representation is displayed to save computer memory. As the user zooms closer to the protein, the representation changes to higher resolution.

When images are used, at a certain point, the 2D image is replaced by a 3D representation of the protein, either the final atomic detail representation, or a reduced 3D representation (see figures); this transition from a simple image to a 3D structure can be arranged so that it is not noticed by the user.

The final 3D representation of the biological element can be rotated in three dimesnions, to provide the user with an insight into the protein three dimensional structure and function; The structure may additionally be annotated with important functional aspects.

The different levels of resolution are included so that the viewer program only needs to download the detailed representation for the proteins that the user is interested in, thus reducing the RAM requirements of the viewer used in the invention.

By carefully choosing the transitions from low resolution to high resolution images, or from 2D image to 3D representation, the user does not notice the transitions, and the zooming appears to be seamless and continuous.

In one session, the user may visit only a relatively small number of protein structure at atomic detail (such as shown in the figures); these structures are cached in RAM and on a local hard disk so they can be quickly revisited.

Unlike other database visualization tools that place proteins into abstract 2D spaces, the view created by the invention is physically and biologically meaningful. Thus each protein is placed into a meaningful context, defined by its subcellular location, and also by the neighboring proteins, DNA, or membranes. This context aids the user in gaining insight into the protein function.

### EXAMPLE 5:

### Selection of data subsets

The method also allows for the possibility to select subsets of the initially displayed data. In the preferred embodiment of the invention, a hierarchical viewer is provided that allows the user to select groups of biological elements by their sub-cellular location (see bottom right panel, Figure 10). Thus clicking on the term 'nucleus' would immediately select all biological elements located in the nucleus. The selected proteins can be indicated by changing their coloring or some other visual means.

Selecting a sub-cellular location can also be facilitated by, for example, double clicking anywhere within a given location.

In the preferred embodiment of the method, an alternative mechanism for enabling the user to define selections is defined. This alternative is a command line (see bottom left panel, Figure 10) where the user can type a query term, for example 'cancer'. This would them select the subset of biological elements currently displayed that are associated with the keyword 'cancer'. The result of query can be found by sending the query term to a meta-database engine, such as SRS,.

### EXAMPLE 6:

### Communication with other applications

In one embodiment of the method, the subsets that are defined by the above methods can be communicated to other computer applications. Also, the method and apparatus according to the invention can receive a list that specifies a subset of proteins (biological elements) to display from another application. As one example, the invention may communicate with an application such as arraySCOUT that analyses data coming from the expression level of many different proteins. With this communication, the user can first select in the in the apparatus according to the einvention only nuclear proteins. This selection can then be sent to the arraySCOUT program, so that only nuclear proteins are selected for the analysis in arraySCOUT. The analysis may then suggest that a given subset of those proteins are of significant interest. The user can then send this subset back to the apparatus according to the invention to see which of these proteins are, for example, complexed with a given chromosome, or which of these proteins occur freely in the nucleus or are complexed with one another.

### EXAMPLE 7:

The method according to the invention can be extended to display different cell types, such as neurons, plant cells, bacteria cells, and viruses or, e.g. different stages of the cell cycle. Additionally, the distribution of biological elements throughout the cell cycle may be shown.

Hereby a "depository" or database is created containing information which may be extracted for creating different types of basic environment elements. One may for example envision also the display of two or more "comparative" basic environment elements ("BEE"), wherein one BEE shows the distribution of biological elements in a (i) normal cell and the other shows the distribution and/or presence within a (ii) diseased cell. One may also envision the subtraction of (i) from (ii) or vice versa and thus merely the display of the remainder. All of the above has enormous advantage of putting the scientist in the position to "grasp" a large amount of information within a single view.

The selection methods can then also be extended to allow the user to choose between the different cell types. In the preferred embodiment, the hierarchy viewer (bottom right panel, Figure 10) shows different organisms; under each organism can be listed each organ; under each organ can be listed each cell type; under each cell type is listed the different sub-cellular locations. The user can select an organism, organ, and cell type, causing the display to switch to the appropriate cell view, and the select of biological elements to switch to those belonging to that cell type.

The inter-application communication can also take advantage of this feature of the invention. For example, the selection of subsets of biological elements from the meta-database engine (e.g. SRS) can be made to depend automatically on the cell type, organ, and organism chosen. Similarly, the selection of organ, organism, and cell type can be communicated to and from applications such as arraySCOUT.

### FURTHER EXAMPLES:

The process of locating at least one of said graphical representations of said biological elements on said display of said basic environment element, can be extended by making use of other types of data sets or databases, such as metabolic or signaling pathway data or protein-protein interaction.

The basic environment element may also be a 3D display., Tthis can be visualized either using a standard PC monitor plus stereo glasses, or with 'immersion'-graphics systems.

The invention can be extended to include dynamics: this will facilitate the visualization of reaction data as well, for example letting the proteins move or 'diffuse' to different locations.

Then, the invention could incorporate yet another type of databases (reactions or metabolic pathways) as source for feature elements.

Data obtained from expression profiles analysis (DNA-chips or protein chip experiments) can also be included using static or dynamic methods.

Processes such as protein expression and/or translocation, or signal transduction, can also be visualized in the invention.

A clear extension is to connect the display of the basic environment element to a viewer of a tissue and/or organism, *i.e*. to see the cells in their relationship to each other, each tissue type, and then the distribution of tissues within the body.

The method according to the invention can be extended to view other macromolecules such as lipid aggregates and RNA; small molecules, such as ATP, can also be incorporated.

### FIGURE CAPTIONS:

FIG. 1:

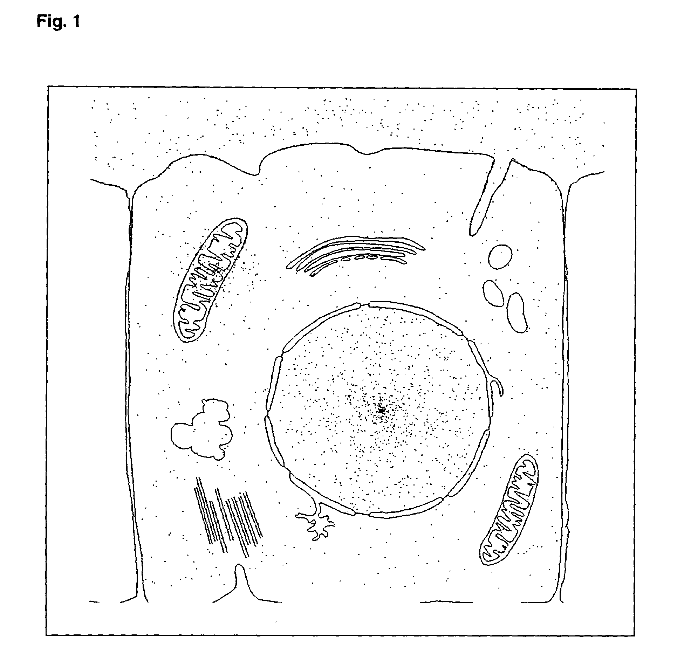

   Figure 1 shows a preferred embodiment of the invention in which a method for organizing and depicting one or more biological elements is performed in such a way that the basic environment element is a generalized eukaryotic cell. The area displayed is about 20x20 µm. One can see that several thousand biological elements have been placed within the "cell" based on information from the various data-sets. One can see that some are also located outside the cell. The representation is a simplification of the preferred embodiment whereby the cell image is generated based on micrograph pictures
FIG. 2:
   Figure 2 A shows a preferred embodiment of the invention whereby the user has chosen the area that is depicted in said display of said basic environment element by choosing an area in a second display of said basic environment element. The user has zoomed into a location within the cell by choosing an area in a first display (2 A) which is then enhanced in a second display of said basic environment element (2B). The user can see e.g. which proteins are located on or in the nuclear membrane. The area shown in 2A is about 10x10 µm in size. The area shown in 2B is 2x2 µm in size. In a preferred embodiment the user may zoom from the area shown in 2A to the area in 2B. In another embodiment two separate displays are chosen.
Fig. 3 to 4 :
   The figures show how the user may change displays. What should be noted is that the various areas displayed may require different kinds of feature elements to be extracted from the various data-sets. In these embodiments for example (3B) protein structures are shown. Thus, when zooming from the area shown in Fig. 1 to the area shown in Fig. 4 a) the number of biological elements that are displayed is reduced drastically however different additional and/or different feature elements are determined in order to, e.g. show the protein structure of a biological element.
FIG. 5:
   Figure 5 shows a typical database entry stemming from the National Institute of Health (USA), pertaining to a nucleic acid molecule.
FIG. 6:

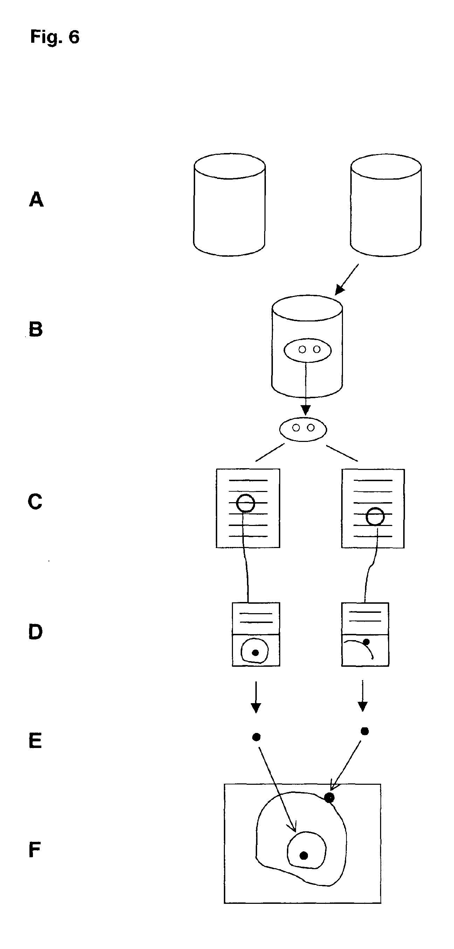

   Figure 6 shows a method for organizing and depicting one or more biological elements comprising the steps of, receiving an input to select one or more databases (A), receiving further input to select one or more desired data sets from the one or more databases based on one or more selection criteria (B), determining at least one first feature element from each of said one or more data sets (C-D), determining a graphical representation for displaying at least one of the biological elements corresponding to said one or more data sets and (E), enhancing a display of a basic environment element by locating at least one of said graphical representations of said biological elements on said display of said basic environment element, depending on the information contained in said first feature element (F). Here, a database was identified which contains data pertaining to two proteins. The data sets were extracted and the entries analyzed, in order to identify information pertaining to their localization in a cell. For each of the proteins a graphical representation was created. The graphical representations of the proteins were placed into the virtual cell based on the information stemming from the data sets. One can see that one protein is located in the nucleus, whereas the other protein is located in the outer cell membrane.
FIG. 7:

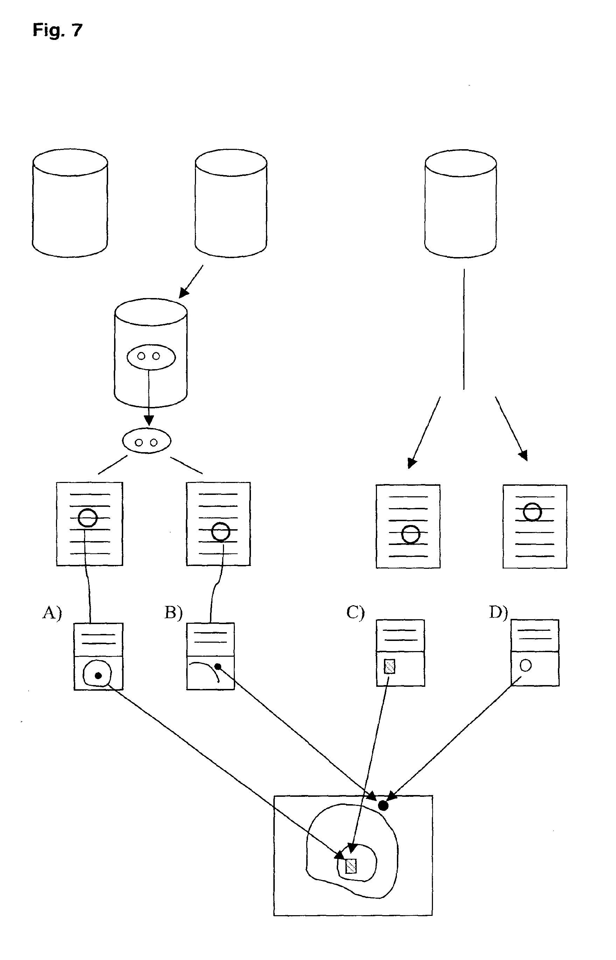

   Figure 7 shows a method for organizing and depicting one or more biological elements comprising the steps of, receiving an input to select one or more databases, receiving further input to select one or more desired data sets from the one or more databases based on one or more selection criteria (A,B), determining at least one first feature element from each of said one or more data sets, determining a graphical representation for displaying at least one of the biological elements corresponding to said one or more data sets and, enhancing a display of a basic environment element by locating at least one of said graphical representations of said biological elements on said display of said basic environment element, depending on the information contained in said first feature element. In this embodiment of the invention a further database is chosen, further feature elements are extracted from data sets therefrom and the feature elements are used to further enhance the display of said at least one graphical representation of said biological element by using the information from said further feature element.
   Here, a database was identified which contains data pertaining to two proteins (A, B). The data sets were extracted and the entries analyzed, in order to identify information pertaining to their localization in a cell. For each of the proteins a graphical representation was created based on data from another database (C, D).
   The graphical representations of the proteins were placed into the virtual cell based on the information stemming from the data sets (A, B). One can see that one protein is located in the nucleus, whereas the other protein is located in the outer cell membrane. Their respective visualization is based on data stemming from data sets C and D.
Fig. 8:

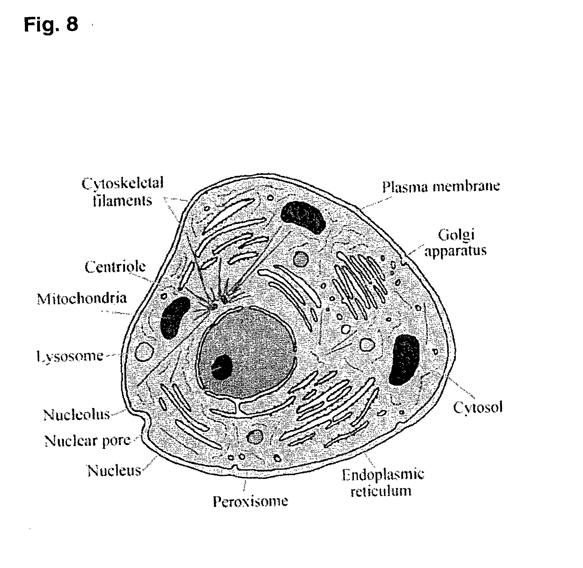

   Figure 8 shows a schematic picture of a eukaryotic cell as it be used to form the basic environment display.
Fig. 9:

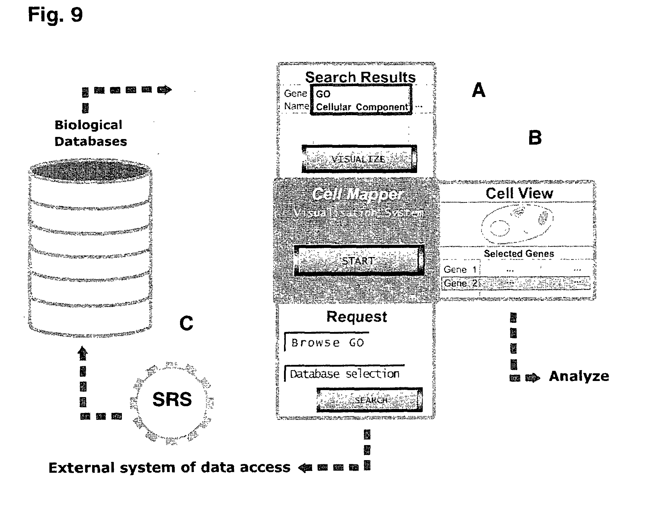

   Figure 9 shows a screenshot from a schematic picture of how one of the embodiments of the method according to the invention is organized. Wherein (B) would be the display, displaying the genes (biological elements) chosen, (A) would be a window allowing the definition of the selection criteria, (C) would be one possible tool that would enable the querying of the databases. In this case SRS (Sequence Retrieval System, see also WO0041094) is used for accessing the databases.
Fig. 10:
   Figure 10 shows one embodiment of the invention wherein a user may select the biological elements to be displayed by choosing these from a list seen in a second "window".
FIG. 11:

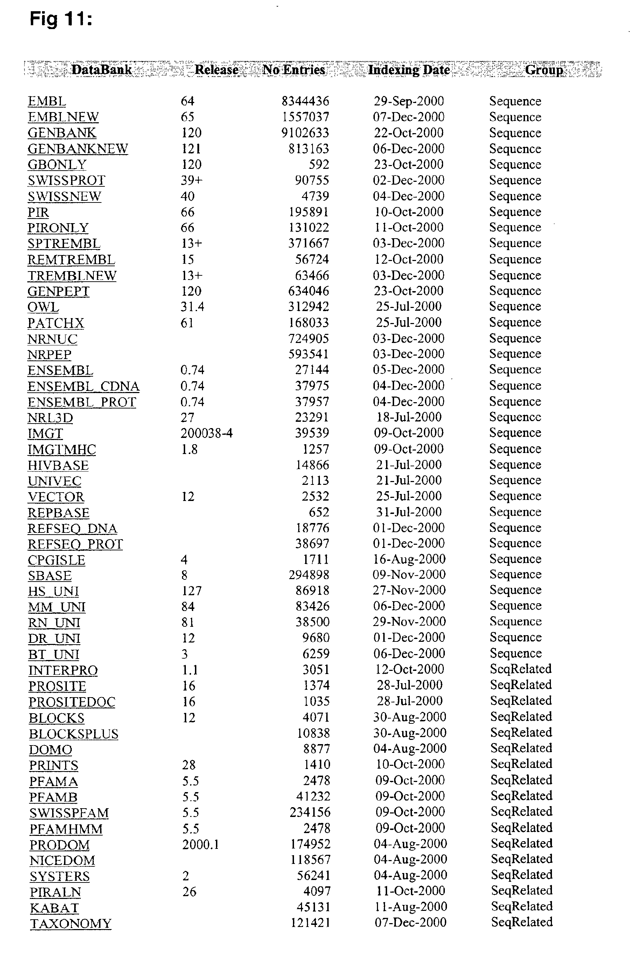

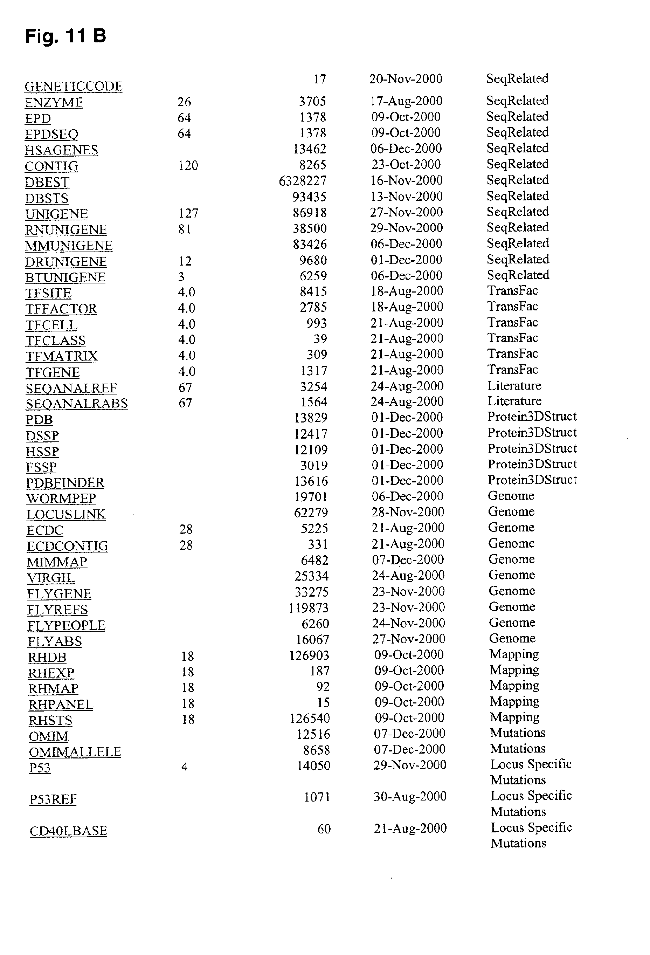

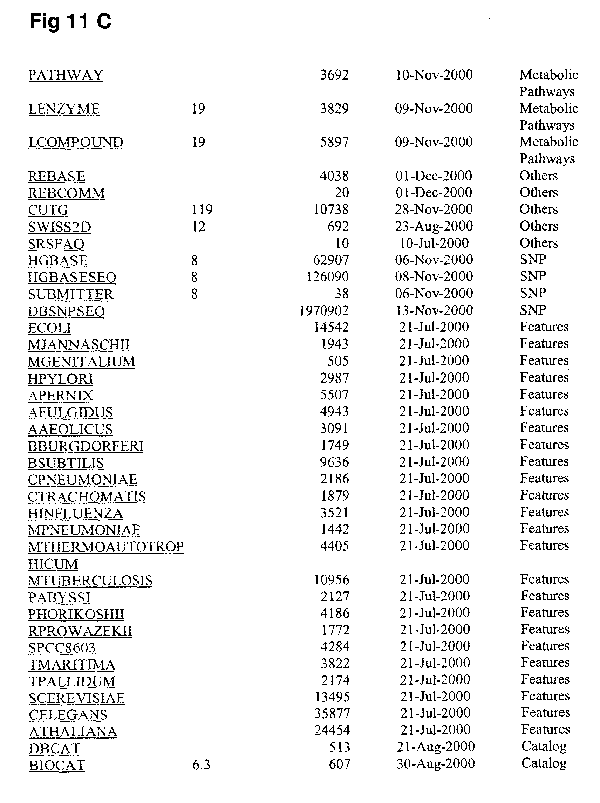

   The figures 11 to 11C show a selection of databases which may be accessed when extracting data sets both for the biological elements as well as for the display of the basic environment element. The figures also show, e.g. what kind of data is contained, how many releases exist, as well as how many entries are contained.

## Claims

1. Method for organizing and depicting one or more biological elements comprising the steps of,
a) receiving an input to select one or more databases,
b) receiving further input to select one or more desired data sets from the one or more databases based on one or more selection criteria,
c) determining at least one first feature element from each of said one or more data sets,
d) determining a graphical representation for displaying at least one of the biological elements corresponding to said one or more data sets and,
e) enhancing a display of a basic environment element by locating at least one of said graphical representations of said biological elements on said display of said basic environment element, depending on the information contained in said first feature element.

2. Method according to claim 1, wherein the biological elements are selected from the group comprising molecules, complexes of molecules, atoms, and/or subatomic particles.

3. Method for organizing and depicting one or more biological elements according to claim 1 or 2, wherein
the display of said basic environment element is enhanced by locating at least 50 of said graphical representations of said biological elements on said display of said basic environment element.

4. Method for organizing and depicting one or more biological elements according to claim 1 or 2, wherein
the display of said basic environment element is enhanced by locating at least 500 of said graphical representations of said biological elements on said display of said basic environment element.

5. Method for organizing and depicting one or more biological elements according to claim 1 or 2, wherein
the display of said basic environment element is enhanced by locating at least 5000 of said graphical representations of said biological elements on said display of said basic environment element.

6. Method according to claims 1 to 5, wherein
one or more further feature elements are determined for said one or more data sets and the one or more further feature elements are extracted from the same database as the database from which the first feature element was determined.

7. Method according to claims 1 to 5, wherein
one or more further feature elements are determined for said one or more data sets and the one or more further feature elements are extracted from one or more different databases as the database from which the first feature element was determined.

8. Method according to claim 6 or 7 comprising the step of,
a) determining at least one second feature element for said at least one data set and,
b) further enhancing the display of said basic environment element by using the information from said second feature element to place the at least one biological element into said basic graphical representation of said basic environment element.

9. Method according to claim 6 or 7 comprising the step of,
a) determining at least one further feature element for said at least one data set and,
b) further enhancing the display of said at least one graphical representations of said biological element by using the information from said further feature element.

10. Method according to claim 9 wherein,
a further feature element for said at least one data set pertaining to said at least one biological element contains information pertaining to one of the activities of said biological elements and the feature element is used to enhance the display of said at least one graphical representation of said biological element by displaying the activity thereof.

11. Method according to any of the above claims, wherein
said one or more databases, are chosen from the group comprising databases that comprise data sets with information regarding biomolecules, organic molecules and/or inorganic molecules found in living organisms.

12. Method according to claim 11, wherein
the databases comprise data sets with information regarding genes and/or proteins.

13. Method according to any of the above claims wherein,
basic environment element is a representation chosen from the group comprising an organism, one or more tissue types, an organ, a cell, an organelle, a sub-cellular compartment, a complex of molecules, a molecule, an atom and/or a sub atomic particle.

14. Method according to claim 13, wherein
the representation is a three dimensional representation.

15. Method according to any of the above claims comprising the step of,
determining the area that is depicted in said display of said basic environment element by selecting an area in a second display of said basic environment element.

16. Data structure representing a graphic display, said data structure being obtained by a method according to one of claims 1 to 15.

17. Data structure according to claim 16, wherein at least 50 of said graphical representations of said biological elements are present.

18. Data structure according to claim 17, wherein at least 500 of said graphical representations of said biological elements are present.

19. Data structure according to claim 18, wherein at least 5000 of said graphical representations of said biological elements are present.

20. A computer readable medium for embodying or storing therein data readable by a computer, said medium comprising one or more of the following:
a) a data structure generated by executing a process according to any of claims 1 to 15;
b) computer program code means which is adapted to cause a computer to execute a method according to any one of claims 1 to 15

21. Apparatus for organizing and depicting one or more biological elements, said apparatus comprising:
f) a receiving module for receiving an input to select one or more databases,
g) a receiving module for receiving further input to select one or more desired data sets from the one or more databases based on one or more selection criteria,
h) a determination module for determining at least one first feature element from each of said one or more data sets,
i) a determination module for determining a graphical representation for displaying at least one of the biological elements corresponding to said one or more data sets and,
j) an enhancing module for enhancing a display of a basic environment element by locating at least one of said graphical representations of said biological elements on said display of said basic environment element, depending on the information contained in said first feature element.
